# EUROPÄISCHE PATENTSCHRIFT

(11) **EP 0 778 032 B1**
(45) Veröffentlichungstag und Bekanntmachung des Hinweises auf die Patenterteilung: **12.02.2003**
(21) Anmeldenummer: 96119250.7
(22) Anmeldetag: 30.11.1996
(51) Int. Cl.: A61M 1/34, A61M 1/36

(54) **Vorrichtung zur Entfernung von toxischen Stoffen aus Blut**
Device for the removal of toxic substances from blood
Dispositif d'élimination des substances toxiques du sang

(30) Priorität: 09.12.1995 DE 19546027
(43) Veröffentlichungstag der Anmeldung: 11.06.1997
(73) Patentinhaber: Fresenius Medical Care Deutschland GmbH, 61352 Bad Homburg v.d.H. (DE)
(72) Erfinder: Beden, Josef, 55252 Mainz-Kastel (DE); Flaig, Hans-Jürgen, Dr., 36341 Lauterbach (DE); Steinbach, Bernd, Dr., 61169 Friedberg (DE)
(74) Vertreter: Luderschmidt, Schüler & Partner GbR

(56) Entgegenhaltungen:
- EP-A- 0 001 074
- EP-A- 0 226 720
- EP-A- 0 321 754
- DE-C- 4 116 178

## Beschreibung

Die Erfindung betrifft eine Vorrichtung zur Entfernung von toxischen Stoffen aus Blut nach dem Oberbegriff des Patentanspruchs 1.

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) überwiegt der diffusive Stofftransport, bei der Hämofiltration (HF) liegt ein konvektiver Stofftransport über die Membran vor. Eine Kombination aus beiden Verfahren ist die Hämodiafiltration (HDF). Bei der Peritonealdialyse (PD) wird kein extrakorporaler Kreislauf benötigt und das Peritoneum als Kontaktmembran ausgenützt.

Wegen der großen Austauschmengen besteht bei den genannten Verfahren, sowie auch bei der kontinuierlichen arterio-venösen HF, der kontinuierlichen veno-venösen HF und der Plasmafiltration (PF) die Notwendigkeit der exakten Bilanzierung von entzogener Flüssigkeit einerseits zur zugeführten Flüssigkeit andererseits und der zu ultrafiltrierenden Menge über die gesamte Behandlungszeit. Zum Stand der Technik gehören gravimetrische und volumetrische Bilanziersysteme.

DE 41 16 178 C1 beschreibt eine Vorrichtung zur Reinigung von Blut mit volumetrischer Flüssigkeitsbilanzierung für HF-, HDF- und PF-Systeme. Die bekannte Blutbehandlungsvorrichtung weist eine Bilanzierkammer auf, die durch eine flexible Wand in eine erste und eine zweite Bilanzierkammerhälfte geteilt ist, wobei die erste Kammerhälfte mit einer Filtratleitung und die zweite Kammerhälfte mit einer Substituatleitung verbunden ist. Von der ersten Kammerhälfte geht eine Filtratauslaßleitung zu einem Auslaß ab, während von der zweiten Kammerhälfte eine Substituatauslaßleitung abgeht, die mit dem Blutkreislauf verbunden ist. Filtrat und Substituat werden der Bilanzierkammer wechselweise jeweils aus einer in die Filtrat- bzw. Substituatleitung geschaltete Ausgleichskammer mittels einer Druckeinrichtung zugeführt. Die Ausgleichskammern werden bei der bekannten Filtrationseinrichtung mit einer Filtratpumpe bzw. einer Substituatpumpe gefüllt und nach Öffnen von Absperrklemmen mittels der Druckeinrichtung in die erste bzw. zweite Bianzierkammerhälfte entleert. Die Absperrklemmen sind dabei so geschaltet, daß bei zulaufendem Filtrat das Substituat abläuft, während bei zulaufendem Substituat das Filtrat abläuft. Demzufolge werden wechselweise zwei Klemmenpaare in die jeweils andere Betriebslage umgeschaltet. Nachteilig ist, daß bei der Verwendung nur eines Bilanziervolumens ein diskontinuierlicher Fluß auftritt, der zur Pulsation führt.

Bei Verwendung einer Bilanziereinrichtung in der PD gilt das Prinzip entsprechend. Hierbei wird jedoch nicht kontinuierlich Substituat zu Filtrat bilanziert, sondern durch zusätzliche Klemmen und Verbindungen zuerst frisches Dialysat über beide Bilanzierkammerhälften gefördert und nach Verweilen im Bauchraum das verbrauchte Dialysat in gleicher Weise in den Abfluß gefördert, d.h. es erfolgt keine unmittelbare Verdrängung von Substituat durch Filtrat und umgekehrt. Die Mengenbilanz erfolgt dabei über Addition der Einzelmengen, die pro Kammerfüllung ab- bzw. zugeführt werden.

Die DE 39 18 078 C2 beschreibt eine Vorrichtung zur Einnadel-Dialyse, bei der während einer arteriellen Saugphase ein Strom unbehandelten Blutes vom Patienten in die arterielle Leitung und während einer venösen Rückführphase ein Strom behandelten Blutes durch die venöse Leitung zu dem Patienten geleitet wird. Es wird vorgeschlagen, stromauf und stromab des Dialysators eine Ausdehnungskammer vorzusehen und die Vorrichtung zur Einnadel-Dialyse in vier aufeinanderfolgenden Phasen zu betreiben, nämlich der Saugphase, einer ersten Übergangsphase, der Rücklaufphase und einer zweiten Übergangsphase. Während der Saugphase werden die stromauf und stromab des Dialysators angeordneten Kammern gleichzeitig gefüllt und während der Rücklaufphase gleichzeitig entleert.

Der Erfindung liegt die Aufgabe zugrunde, eine Vorrichtung zur Entfernung von toxischen Stoffen aus Blut mit volumetrischer Flüssigkeitsbilanzierung zu schaffen, bei der das Auftreten eines pulsativen Flusses weitgehend vermieden wird.

Bei der erfindungsgemäßen Vorrichtung zur Entfernung von toxischen Stoffen aus Blut gemäß Anspruch ist eine Pufferkammer variablen Volumens in die erste und/oder zweite Auslaßleitung geschaltet. Für den Fall, daß die Pufferkammer in die erste Auslaßleitung geschaltet ist, wird das Zusatzvolumen immer genau dann bereitgestellt, wenn die zweite Ausgleichskammer in die zweite Bilanzierkammerhälfte unter Verdrängung der Flüssigkeit aus der ersten Bilanzierkammerhälfte entleert wird. Dadurch kann sich die Pufferkammer mit Flüssigkeit füllen. Wenn hingegen Flüssigkeit in die zweite Ausgleichskammer fließt, wird das Volumen der Pufferkammer verringert, so daß die darin befindliche Flüssigkeit langsam ausströmt und eine Glättung des diskontinuierlich erfolgenden Pulses erreicht wird. Für den Fall, daß die Pufferkammer in die zweite Auslaßleitung geschaltet ist, wird das Zusatzvolumen immer genau dann bereitgestellt, wenn die erste Ausgleichskammer in die erste Bilanzierkammerhälfte unter Verdrängung der Flüssigkeit aus der zweiten Bilanzierkammerhälfte entleert wird. Zur Glättung des diskontinuierlich erfolgenden Pulses können aber auch in beiden Auslaßleitungen der Bilanzierkammer jeweils eine Pufferkammer vorgesehen sein.

Prinzipiell ist es möglich, das Volumen der Pufferkammer schon unmittelbar vor dem Entleeren der ersten bzw. zweiten Ausgleichskammer bereitzustellen. Vorzugsweise wird das Volumen der Pufferkammer aber beim Entleeren der ersten bzw. zweiten Ausgleichskammer allmählich vergrößert. Für den Fall, daß das maximale Volumen der Pufferkammer geringer als das Volumen der jeweiligen Ausgleichskammer ist oder das Volumen der Pufferkammer langsamer vergrößert wird als das Volumen der Ausgleichskammer abnimmt, kann sichergestellt werden, daß auch während des Befüllungsvorganges der Pufferkammer immer Flüssigkeit in die Auslaßleitung strömt.

Zweckmäßigerweise sind die erste und zweite Ausgleichskammer mittels Kompressionseinrichtungen mit Druck beaufschlagt, so daß sich diese beim Öffnen der Klemmeinrichtungen selbständig in die jeweilige Bilanzierkammerhälfte entleeren.

In einer bevorzugten Ausführungsform weist die Kompressionseinrichtung der ersten und zweiten Ausgleichskammer jeweils zwei durch ein Federelement zusammengedrückte Druckelemente auf, die die Kammer über- bzw. untergreifen. Ferner sind zwei die Pufferkammer unter- bzw. übergreifende Druckelemente vorgesehen, wobei die Druckelemente der in die erste bzw. zweite Auslaßleitung geschalteten Pufferkammer und der zweiten bzw. ersten Ausgleichskammer derart miteinander gekoppelt sind, daß diese eine gegenläufige Bewegung ausüben. Dadurch wird erreicht, daß das Volumen der Pufferkammer beim Entleeren der jeweiligen Ausgleichskammer vergrößert und beim Befüllen der jeweiligen Ausgleichskammer verringert wird.

In einer besonders einfachen und störungsunanfälligen Ausführungsform erfolgt die Kopplung der Druckelemente auf mechanische Weise mittels einer Wippe. Eines der beiden Druckelemente der Ausgleichskammer bzw. der Pufferkammer ist hierzu ortsfest angeordnet, während das andere der beiden Druckelemente verschiebbar geführt ist. Die verschiebbar geführten Druckelemente sind über ein Verbindungselement gekoppelt, das zwischen seinen Enden schwenkbar gelagert ist. Durch Veränderung des Hebelverhältnisses kann das Maß der Veränderung des Volumens der Pufferkammer eingestellt werden, so daß sich eine optimale Glättung erreichen läßt.

Da die Gesamtmenge des Fluids in einem Zyklus wegen der Zwischenspeicherung in der Pufferkammer nicht auf einmal gefördert wird, dadurch der Gegendruck reduziert wird und sich der Druck automatisch mittels der Kopplung auf die jeweilige Ausgleichskammer überträgt, kann die benötigte Federkraft der Kompressionseinrichtung der jeweiligen Ausgleichskammer reduziert werden.

Nachfolgend wird unter Bezugnahme auf die Zeichnungen ein Ausführungsbeispiel der Erfindung näher erläutert.

Es zeigen:
- Fig. 1: eine schematische Darstellung einer Blutreinigungsvorrichtung mit einer Filtrat/Substituat-Bilanziereinrichtung,
- Fig. 2: ein Disposable mit einer Bilanzierkammer, einer ersten Ausgleichskammer, einer zweiten Ausgleichskammer und einer Pufferkammer in der Draufsicht,
- Fig. 3: einen Schnitt durch die Bilanzierkammer des Disposables von Fig. 2,
- Fig. 4: den Systemeinschub der Blutreinigungsvorrichtung zur Aufnahme des Disposables in der Draufsicht und
- Fig. 5: eine Ansicht des Systemeinschubs aus der Richtung des Pfeils V von Fig. 4.

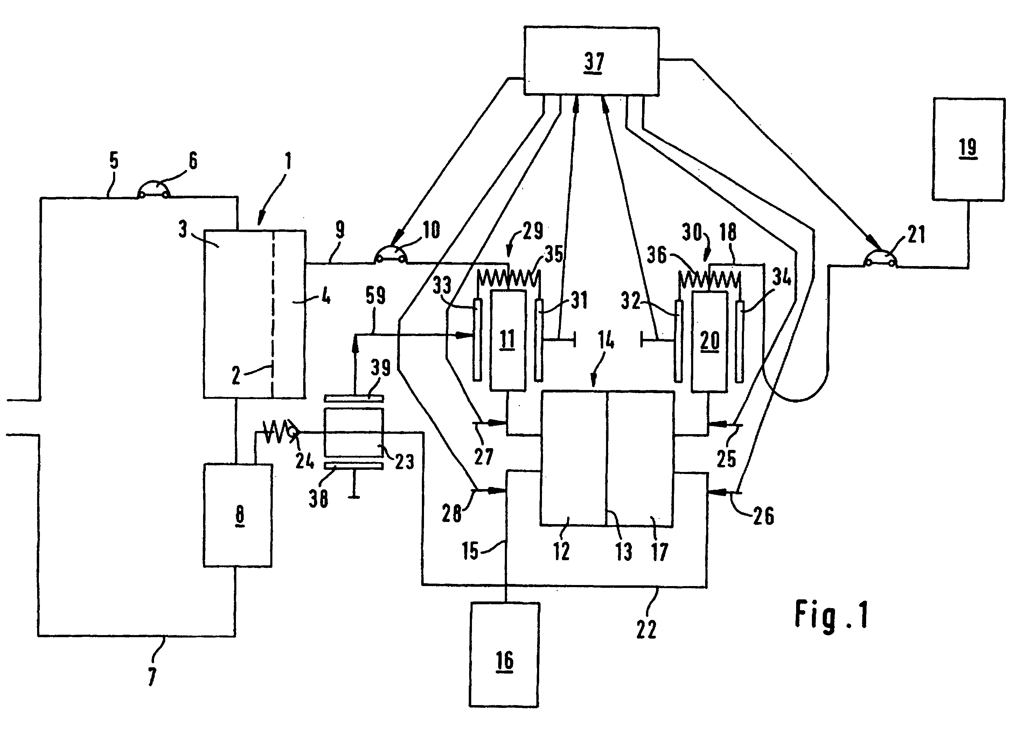

Fig. 1 zeigt eine Ausführungsform der Vorrichtung zur Entfernung von toxischen Stoffen aus Blut, in der die erfindungsgemäße Bilanziereinrichtung in vorteilhafter Weise Verwendung finden kann. Das Grundprinzip der Filtrationsvorrichtung ist aus DE 41 16 178 C1 bekannt, auf die ausdrücklich Bezug genommen wird. Die Filtrationsvorrichtung weist einen Filter 1 auf, der durch eine semipermeable Membran 2 in eine Blutkammer 3 und eine Filtratkammer 4 geteilt ist. Die Blutkammer 3 ist an ihrem Einlauf mit einer zum Patienten führenden Blutzuführleitung 5 verbunden, in die eine Blutpumpe 6 geschaltet ist. In der zum Patienten führenden Blutabflußleitung 7 ist eine Tropfkammer 8 geschaltet.

Auf der Filtratseite ist die Filtratkammer 4 über eine erste Einlaßleitung 9, in die eine erste Pumpe 10 und eine erste Ausgleichskammer 11 geschaltet ist, mit dem Einlaß der ersten Bilanzierkammerhälfte 12 einer durch eine flexible Wand 13 in zwei Bilanzierkammerhälften geteilten Bilanzierkammer 14 verbunden. Vom Auslaß der ersten Bilanzierkammerhälfte 12 geht eine erste Auslaßleitung 15 zum Ausguß 16 ab.

Der Einlaß der zweiten Bilanzierkammerhälfte 17 ist über eine zweite Einlaßleitung 18 mit einer Substituatquelle 19 verbunden. In die zweite Einlaßleitung 18 ist eine zweite Ausgleichskammer 20 und eine zweite Pumpe 21 geschaltet. Vom Auslaß der zweiten Bilanzierkammerhälfte 17 geht eine zweite Auslaßleitung 22 zu der venösen Tropfkammer 8 ab. In die zweite Auslaßleitung 22 ist eine Pufferkammer 23 geschaltet. Stromab der Pufferkammer 23 befindet sich in der zweiten Auslaßleitung 22 ein Rückschlagventil 24.

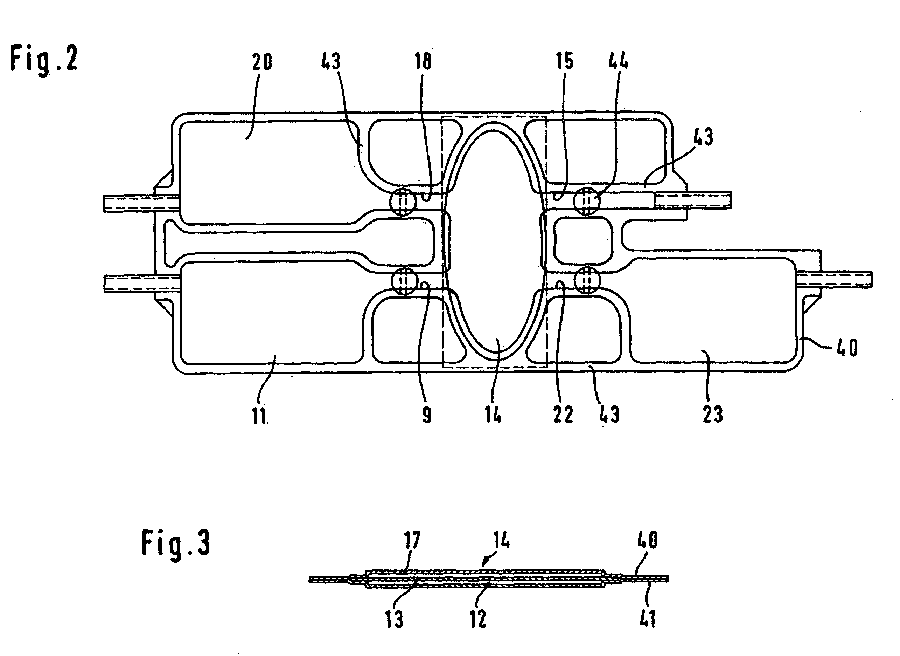

Die erste Einlaßleitung 9 und die erste Auslaßleitung 15 sowie die zweite Einlaßleitung 18 und die zweite Auslaßleitung 22 sind mittels Klemmeinrichtungen 25 bis 28 abklemmbar. Die Bilanzkammer 14, die erste Ausgleichskammer 11, die zweite Ausgleichskammer 20 und die Pufferkammer 23 sind als flexible Kunststoffbeutel ausgebildet und sind Bestandteil eines Foliendisposables, das unter Bezugnahme auf die Fign. 2 und 3 noch im einzelnen beschrieben wird.

Die erste Ausgleichskammer 11 und die zweite Ausgleichskammer 20 sind jeweils mit einer Kompressionseinrichtung 29, 30 versehen, die jeweils aus einer ortsfesten Druckplatte 31, 32 und einer verschiebbar geführten Druckplatte 33, 34 bestehen, die über ein Federelement 35 bzw. 36 zusammengedrückt sind. Die Druckplattenpaare 31, 33 bzw. 32, 34 über- bzw. untergreifen die Ausgleichskammern 11, 20 und entleeren diese im Entleerungsschritt über die Zuleitungen 9, 18 in die Bilanzierkammerhälften 12, 17.

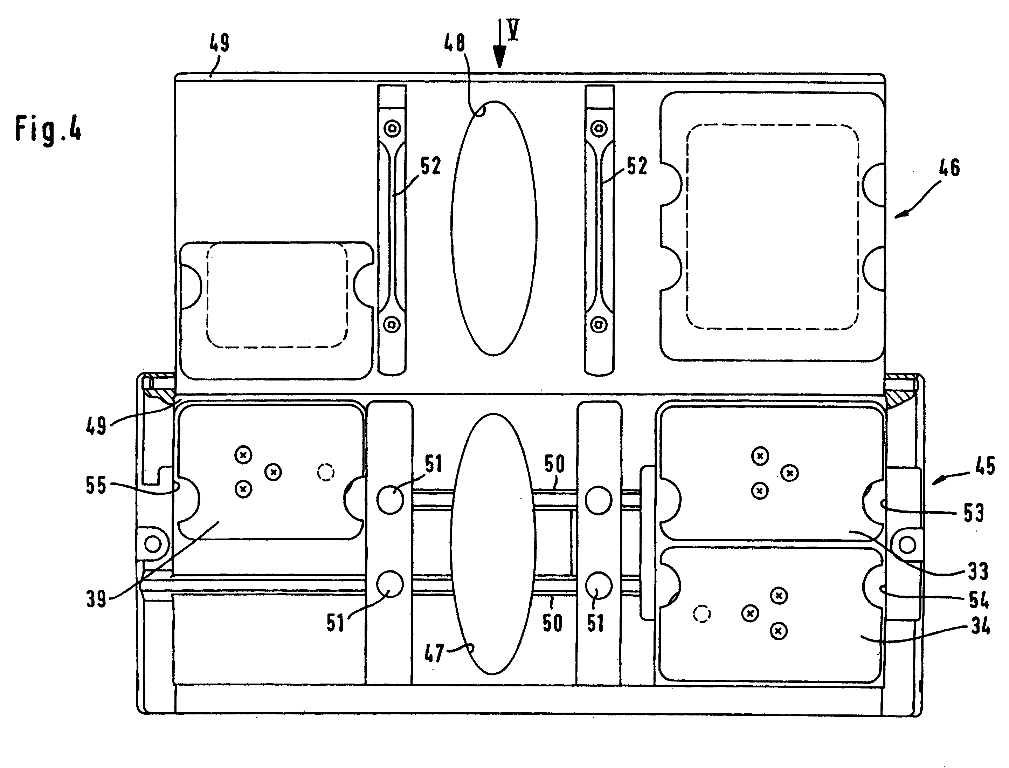

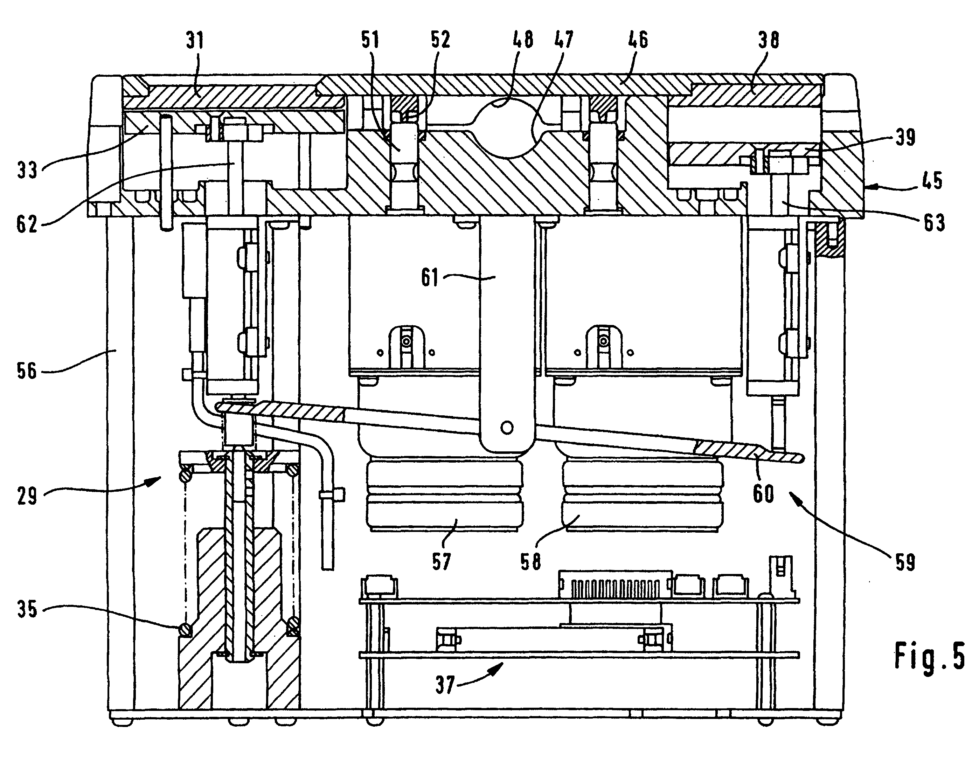

Die erste Pumpe 10 und die zweite Pumpe 21 sowie die Klemmeinrichtungen 25 bis 28 sind über Steuerleitungen mit einer Steuer- und Regeleinheit 37 verbunden. An der Pufferkammer 23 greifen ferner zwei Druckplatten 38, 39 an, wobei die eine Druckplatte 38 ortsfest und die andere Druckplatte 39 verschiebbar geführt ist. Die verschiebbar geführte Druckplatte 39 der Pufferkammer 23 ist mit der verschiebbar geführten Druckplatte 33 der ersten Ausgleichskammer 11 über eine im einzelnen noch unter Bezugnahme auf die Fign. 4 und 5 beschriebenen mechanischen Kopplung verbunden. Die Druckplatten 33, 39 sind derart gekoppelt, daß diese eine gegenläufige Bewegung ausüben.

Fig. 2 zeigt das Bilanzier-Foliendisposable mit der Bilanzierkammer 14, der ersten Ausgleichskammer 11, der zweiten Ausgleichskammer 20 und der Pufferkammer 23 in der Draufsicht. Das Foliendisposable weist zwei flexibel im wesentlichen rechteckförmige Kunststoffolien 40, 41 auf, die entlang der mit den Bezugszeichen 43 versehenen Nahtstellen derart miteinander verschweißt sind, daß zwischen der oberen und unteren Folie 40, 41 vier Kammern ausgebildet sind. Im Zentrum befindet sich die Bilanzierkammer 14 mit einer ovalen Form, wobei auf der einen Seite der Bilanzierkammer 14 die erste Ausgleichskammer 11 und die zweite Ausgleichskammer 20 und auf der anderen Seite die Pufferkammer 23 angeordnet sind. Die mit Flüssigkeit gefüllten Kammern 11, 20, 23 haben annähernd das gleiche Volumen.

Die flexible Wand 13, die die Bilanzierkammer 14 in die erste und zweite Bilanzierkammerhälfte 12, 17 unterteilt, wird durch eine in Fig. 2 mit gestrichelten Linien angedeutete rechteckförmige Zwischenfolie gebildet, die im Bereich des Bilanziervolumens zwischen die obere und untere Folie 40, 41 eingelegt ist und unter Ausbildung der beiden Kammerhälften 12, 17 mit den äußeren Folien 40, 41 verschweißt ist.

Die erste Einlaßleitung 9, die erste Auslaßleitung 15, die zweite Einlaßleitung 18 und die zweite Auslaßleitung 22 sind im Bereich des Bilanzierdisposables als Folienkanäle ausgebildet, in denen in Fig. 2 nicht dargestellte Kunststoffschläuche eingelegt sind, die mit der oberen und unteren Folie 40, 41 verschweißt sind. Die eingelegten Kunststoffschläuche verhindern nicht nur ein Kollabieren der Schlauchkanäle, sondern schaffen auch Klemmstellen 44, die sich druckdicht mittels der Klemmeinrichtungen 25 bis 28 abdichten lassen.

Fig. 3 zeigt einen Schnitt durch die Bilanzierkammer 14 des ungefüllten Bilanzierdisposables. Die Zwischenfolie 13 ist mit ihrem äußeren Rand mit den Rändern der Ober- und Unterfolie 40, 41 verschweißt und in der Bilanzierkammer zwischen den äußeren Folien frei beweglich. Da sich wegen der Flexibilität der Kunststoffolien ein konstantes Volumen nicht genau einhalten läßt, wird das Bilanziervolumen durch eine feste äußere Form definiert. Das Bilanzierdisposable wird daher in einen Systemeinschub eingesetzt, der Bestandteil der Blutreinigungsvorrichtung ist.

Fig. 4 zeigt den aufgeklappten Systemeinschub in der Draufsicht, in den das Bilanzierdiposable passend eingelegt werden kann. Der Systemeinschub weist zwei Aufnahmekörper 45, 46 mit jeweils einer im Bereich des Bilanziervolumens vorgesehenen Vertiefung 47 bzw. 48 auf. Die Vertiefungen 47, 48 der beiden Aufnahmekörper 45, 46 sind im Zentrum der beiden Aufnahmekörper angeordnet. Der obere und untere Aufnahmekörper 45, 46 des Systemeinschubs sind mit einem längslaufenden Klemmrand 49 versehen, der die Randbereiche des Disposables fixiert. Wird das Volumen zwischen der Oberfolie 40 und der Zwischenfolie 13 der Bilanzierkammer 14 mit Flüssigkeit gefüllt, so wird bei entsprechendem Öffnen und Schließen der Ein- und Ausgänge der Kammer 14 durch Umschlagen der Zwischenfolie 13 das andere Volumen zwischen der Zwischenfolie 13 und der Unterfolie 41 infolge der Verdrängung entleert. Damit sich die Ober- und Unterfolie 40, 41 des Disposables dicht an die Schalen der Aufnahmekörper 45, 46 anlegen können, sind in den Aufnahmekörpern Entlüftungsöffnungen vorgesehen. Zu beiden Seiten der Vertiefungen 47, 48 sind in dem unteren Aufnahmekörper 45 jeweils zwei parallele Führungskanäle 50 zur Aufnahme der Ein- und Auslaßkanäle vorgesehen. Ferner sind innerhalb der Führungskanäle vier Stößel 51 angeordnet, die mit Klemmkanten 52 zusammenwirken, die sich auf der Höhe der Stößel in dem oberen Aufnahmekörper 46 befinden. Mit den elektromagnetisch betätigbaren Stößeln 51 können die Klemmstellen 44 der Kanäle des Disposables druckdicht abgeklemmt werden. Seitlich neben den Stößeln 51 bzw. den Klemmkanten 52 sind in dem unteren und oberen Aufnahmekörper 45, 46 Einsatzstücke 53, 54, 55 für die erste und zweite Ausgleichskammer 11, 20 sowie für die Pufferkammer 23 vorgesehen. Am Boden der Einsatzstücke 53, 54 für die erste und zweite Ausgleichskammer 11, 20 sind die verschiebbar geführten und federnd vorgespannten Druckplatten 33, 34 angeordnet, mit denen die Ausgleichskammern 11, 20 des Disposables stetig mit Druck beaufschlagt werden.

Die Druckplatte am Boden des Einsatzstücks 55 der Pufferkammer 23 ist mit der Druckplatte 33 der ersten Ausgleichskammer 11 derart mechanisch gekoppet, daß diese eine gegenläufige Bewegung ausübt, d.h., wenn die verschiebbar geführte Druckplatte 33 der ersten Ausgleichskammer 11 angehoben wird, senkt sich die verschiebbar geführte Druckplatte 39 der Pufferkammer 23 und umgekehrt.

Fig. 5 zeigt den Systemeinschub in der Seitenansicht. Der untere Aufnahmekörper 45 wird von einem Rahmen 56 getragen, der die Antriebselemente 57, 58 für die Stößel 52, die Kompressionseinrichtungen 29, 30 mit den beiden mittels Druckfedern vorgespannten Druckplatten und eine Platine für die Steuerungs- und Regeleinheit 37 aufnimmt. Die mechanische Kopplung der verschiebbar geführten Druckplatte 33 der ersten Ausgleichskammer 11 und der verschiebbar geführten Druckplatte 39 der Pufferkammer 23 erfolgt über eine Wippe 59. Diese weist ein Verbindungselement 60 auf, das an einem Tragelement 61 zwischen seinen beiden Enden in der Mitte schwenkbar gelagert ist und mit seinen Enden an den Führungsstangen 62, 63 der Druckplatten 33, 39 angreift. Wenn die erste Ausgleichskammer 11 befüllt wird und sich die Druckplatte 33 entgegen der Kraft der Druckfeder 35 nach unten gedrückt wird, wird die Druckplatte 39 der Pufferkammer 23 angehoben. Andererseits führt ein Absenken der Druckplatte 39 der Pufferkammer 23 zu einem Anheben der Druckplatte 33 der ersten Ausgleichskammer 11.

Nachfolgend wird der Ablauf der Bilanzierung beschrieben. Zunächst wird die erste Ausgleichskammer 11 mittels der ersten Pumpe 10 befüllt. Nach Öffnen der Klemmeinrichtung 27 in der ersten Einlaßleitung 9 entleert sich die flexible erste Ausgleichskammer 11 unter dem Druck der Druckplatte 33 in die erste Bilanzierkammerhälfte 12. Die einströmende Flüssigkeit verdrängt das in der zweiten Bilanzierkammerhälfte 17 vorhandene Fluid. Beim Entleeren der ersten Ausgleichskammer 11 vergrößert sich das Volumen der Pufferkammer 23 durch Absenken der Druckplatte 39 in dem Maße, wie sich das Volumen der ersten Ausgleichskammer 11 durch Anheben der Druckplatte 33 verringert. Das aus der zweiten Bilanzierkammerhälfte 17 austretende Fluid füllt teilweise die Pufferkammer 23 und fließt dann ab. Nachdem das Bilanziervolumen völlig gefüllt ist, beginnt sich die erste Ausgleichskammer 11 wieder zu füllen und die zweite Ausgleichskammer 20 entleert sich unter Verwerfung des Fluids in das Bilanziervolumen. Beim Füllen der ersten Ausgleichskammer 11 wird die verschiebbar geführte Druckplatte 33 abgesenkt.

Durch die mechanische Kopplung über die Wippe 59 hebt sich gleichzeitig die verschiebbar geführte Druckplatte 39 der Pufferkammer 23, so daß die im Zusatzvolumen befindliche Restflüssigkeit herausgedrückt wird. Dadurch bleibt der Fluß auch beim Befüllen der ersten Ausgleichskammer 11 bestehen, bis im nächsten Takt wieder neue Flüssigkeit aus dem Bilanziervolumen nachfließt. Der dafür notwendige Druck wird durch die Ausdehnung der ersten Ausgleichskammer 11 geliefert. Auf diese Weise wird eine Glättung des diskontinuierlich erfolgenden Pulses erreicht.

Das Rückschlagventil 24 in der zweiten Auslaßleitung 22 stromab der Pufferkammer 23 verhindert in der unter Bezugnahme auf Fig. 1 beschriebenen Ausführungsform einer Vorrichtung zur Entfernung von Giftstoffen aus Blut bei Entwässerungszyklen einen deutlichen Druckabfall. Sobald die erste Ausgleichskammer nämlich entleert wird, gibt diese das zusätzliche Puffervolumen über die Kopplung frei. Bei einem Entwässerungszyklus wird dieses Volumen aber nicht durch das Substituat eingenommen, sondern füllt sich durch den venösen Gegendruck von der Tropfkammer 8 her mit Luft oder Blut, wobei der Druck in der Tropfkammer 8 absinkt. Dieser Druckabfall wird durch das Rückschlagventil 24 im richtigen Augenblick verhindert. Alternativ kann aber auch ein Absperrventil vorgesehen sein, das die Zuleitung zum extrakorporalen Kreislauf bei Bedarf schließt.

Bei der oben beschriebenen Ausführungsform ist ferner zu beachten, daß die Federkraft der Kompressionseinrichtung 30 der zweiten Ausgleichskammer 20 nicht stärker dimensioniert sein darf als die Federkraft der Kompressionseinrichtung 29 der ersten Ausgleichskammer 11, damit die Bilanzierkammer 14 bei Entwässerungszyklen, d.h. bei Zyklen, bei denen eine Filtration ohne Substitution erfolgt, richtig entleert werden kann.

Die eine Bilanzierkammer, Klemmeinrichtungen, Ein- und Auslaßleitungen und mindestens eine Pufferkammer sowie eine Steuereinheit aufweisende Bilanziereinrichtung kann auch in vorteilhafter Weise in einer Vorrichtung zur Peritonealdialyse Verwendung finden, mit der zyklisch Peritonealdialysierflüssigkeit in exakt bilanzierter Weise einem Patienten zugeführt bzw. entnommen werden kann. Hierzu werden die beiden Einlaßleitungen 9, 18 zu einer gemeinsamen Einlaßleitung zusammengeführt und die beiden Auslaßleitungen 15, 22 werden zu einer gemeinsamen Auslaßleitung zusammengeführt, wobei die Dialysierflüssigkeit über die gemeinsame Einlaßleitung wechselweise in die erste und zweite Bilanzierkammerhälfte 12, 17 geleitet wird und über die gemeinsame Auslaßleitung dem Peritonealraum des Patienten zugeführt wird. Bei einem derartigen Peritonealdialysegerät wird vorteilhafterweise eine Pufferkammer in die erste und eine Pufferkammer in die zweite Auslaßleitung geschaltet. Alternativ kann aber auch eine Pufferkammer nach der Zusammenführung in die gemeinsame Auslaßleitung geschaltet werden.

## Patentansprüche

1. Vorrichtung zur Entfernung von toxischen Stoffen aus Blut mit
einer Bilanzierkammer (14), die durch eine flexible Wand (13) in eine erste mit einer ersten Einlaßleitung (9) verbundenen und eine zweite mit einer zweiten Einlaßleitung (18) verbundenen Bilanzierkammerhälfte (12, 17) geteilt ist, wobei von der ersten Bilanzierkammerhälfte (12) eine erste Auslaßleitung (15) abgeht und von der zweiten Bilanzierkammerhälfte (17) eine zweite Auslaßleitung (22) abgeht,
Klemmeinrichtungen (25 bis 28) zum Absperren der Einlaßleitungen (9, 18) und Auslaßleitungen (15, 22),
einer Steuereinheit (37) zum Aktivieren der Klemmeinrichtungen (25 bis 28) und
einer in die erste Einlaßleitung (9) geschalteten ersten Ausgleichskammer (11) und einer in die zweite Einlaßleitung (18) geschalteten zweiten Ausgleichskammer (20),
**dadurch gekennzeichnet,**
**daß** in die erste und/oder zweite Auslaßleitung (15, 22) der Bilanzierkammer (14) eine Pufferkammer (23) geschaltet ist, deren Volumen veränderbar ist, wobei eine Kopplungseinrichtung vorgesehen ist, die derart ausgebildet ist, daß das Volumen der in die erste bzw. zweite Auslaßleitung (15 bzw. 22) geschalteten Pufferkammer (23) in Abhängigkeit von dem Volumen der zweiten bzw. ersten Ausgleichskammer (20 bzw. 11) derart veränderbar ist, daß das Volumen der Pufferkammer unmittelbar vor dem Entleeren oder beim Entleeren der zweiten bzw. ersten Ausgleichskammer vergrößert und beim Befüllen der zweiten bzw. ersten Ausgleichskammer verringert wird.

2. Vorrichtung nach Anspruch 1, **dadurch gekennzeichnet, daß** die erste und zweite Ausgleichskammer (11, 20) jeweils von stetig auf sie Druck ausübenden Kompressionseinrichtungen (29, 30) beaufschlagt sind.

3. Vorrichtung nach Anspruch 2, **dadurch gekennzeichnet, daß** die Kompressionseinrichtungen (29, 30) der ersten und zweiten Ausgleichskammer (11, 20) jeweils zwei durch ein Federelement (35, 36) zusammengedrückte, die Kammer über- bzw. untergreifende Druckelemente (31, 33; 32, 34) aufweisen, und daß zwei Druckelemente (38, 39) vorgesehen sind, welche die in die erste bzw. zweite Auslaßleitung (15 bzw. 22) geschaltete Pufferkammer (23) unter- bzw. übergreifen, wobei die Kopplungseinrichtung (60) die Druckelemente (38, 39) der in die erste bzw. zweite Auslaßleitung (15 bzw. 22) geschalteten Pufferkammer und die Druckelemente (31, 33) der zweiten bzw. ersten Ausgleichskammer (20 bzw. 11) derart koppelt, daß die Druckelemente (38, 39) eine gegenläufige Bewegung ausüben, wodurch das Volumen der in der ersten bzw. zweiten Auslaßleitung (15, 22) geschalteten Pufferkammer (23) beim Entleeren der zweiten bzw. ersten Ausgleichskammer (20 bzw. 11) vergrößert und beim Befüllen der zweiten bzw. ersten Ausgleichskammer verringert wird.

4. Vorrichtung nach Anspruch 3, **dadurch gekennzeichnet, daß** jeweils eine der beiden Druckelemente (31, 32, 38) der ersten und zweiten Ausgleichskammer (11, 20) und der in der ersten bzw. zweiten Auslaßleitung (15, 22) geschalteten Pufferkammer (23) ortsfest angeordnet und die jeweils andere der beiden Druckelemente (33, 34, 39) verschiebbar geführt ist, wobei die Kopplungseinrichtung ein zwischen seinen Enden schwenkbar gelagertes Verbindungselement (60) ist, über das das verschiebbar geführte Druckelement (39) der in der ersten bzw. zweiten Auslaßleitung (15 bzw. 22) geschalteten Pufferkammer (23) und das verschiebbar geführte Druckelement (33) der zweiten bzw. ersten Ausgleichskammer (20, 11) mechanisch miteinander gekoppelt sind.

5. Vorrichtung nach einem der Ansprüche 1 bis 4, **dadurch gekennzeichnet, daß** stromab der in der ersten bzw. zweiten Auslaßleitung (15 bzw. 22) geschalteten Pufferkammer (23) in der ersten bzw. zweiten Auslaßleitung (15, 22) ein Rückschlagventil (24) oder ein Absperrventil vorgesehen ist.

## Revendications

1. Dispositif pour éliminer des substances toxiques du sang, comprenant
une chambre d'équilibrage (14) qui est subdivisée par une paroi flexible (13) en une première moitié de chambre d'équilibrage (12) reliée à un premier conduit d'entrée (9) et en une deuxième moitié de chambre d'équilibrage (17) reliée à un deuxième conduit d'entrée (18), dans lequel, un premier conduit de sortie (15) part de la première moitié de chambre d'équilibrage (12) et un deuxième conduit de sortie (22) part de la deuxième moitié de chambre d'équilibrage (17),
des mécanismes de serrage (25 à 28) pour la fermeture des conduits d'entrée (9, 18) et des conduits de sortie (15, 22),
une unité de commande (37) pour activer les mécanismes de serrage (25 à 28), et
une première chambre de compensation (11) montée dans le premier conduit d'entrée (9) et une deuxième chambre d'équilibrage (20) montée dans le deuxième conduit d'entrée (18),
**caractérisé en ce que**
une chambre tampon (23) est montée dans le premier et/ou le deuxième conduit de sortie (15, 22) de la chambre d'équilibrage (14), dont le volume peut être modifié, un mécanisme d'accouplement étant prévu qui est réalisé de telle sorte que le volume régnant dans la chambre tampon (23) montée dans le premier, respectivement le deuxième conduit de sortie (15, respectivement 22) peut être modifié en fonction du volume de la deuxième, respectivement de la première chambre de compensation (20, respectivement 11) de telle sorte que le volume de la chambre tampon immédiatement avant le vidage ou au cours du vidage de la deuxième, respectivement de la première chambre de compensation augmente et diminue lors du remplissage de la deuxième, respectivement de la première chambre de compensation.

2. Dispositif selon la revendication 1, **caractérisé en ce que** la première et la deuxième chambre de compensation (11, 20) sont respectivement sollicitées par des mécanismes de compression (29, 30) exerçant constamment une pression sur lesdites chambres.

3. Dispositif selon la revendication 2, **caractérisé en ce que** les mécanismes de compression (29, 30) de la première et de la deuxième chambre de compensation (11, 20) présentent respectivement deux éléments de pression (31, 33 ; 32, 34) comprimés par un élément de ressort (35, 36), venant s'appliquer par-dessus et par-dessous les chambres et **en ce qu'**on prévoit deux éléments de pression (38, 39) qui viennent s'appliquer par-dessous respectivement par-dessus la chambre tampon (23) montée dans le premier, respectivement le deuxième conduit de sortie (15, respectivement 22), dans lequel le mécanisme d'accouplement (60) accouple les éléments de pression (38, 39) de la chambre tampon montée dans le premier, respectivement le deuxième conduit de sortie (15, respectivement 22) et les éléments de pression (31, 33) de la deuxième, respectivement de la première chambre de compensation (20, respectivement 11) de telle sorte que les éléments de pression (38, 39) effectuent des mouvements en sens contraire, si bien que le volume régnant dans la chambre tampon (23) montée dans le premier, respectivement le deuxième conduit de sortie (15, 22) augmente lors du vidage de la deuxième, respectivement de la première chambre de compensation (20, respectivement 11) et diminue lors du remplissage de la deuxième, respectivement la première chambre de compensation.

4. Dispositif selon la revendication 3, **caractérisé en ce que**, respectivement, un des deux éléments de pression (31, 32, 38) de la première et de la deuxième chambre de compensation (11, 20) et de la chambre tampon (23) montée dans le premier, respectivement le deuxième conduit de sortie (15, 22) est disposé à demeure et **en ce que**, respectivement l'autre des deux éléments de pression (33, 34, 39) est guidé en coulissement, dans lequel le mécanisme d'accouplement représente un élément de liaison (60) monté en pivotement entre ses extrémités par lequel, l'élément de pression (39) guidé en coulissement de la chambre tampon (23) montée dans le premier, respectivement le deuxième conduit de sortie (15, respectivement 22) et l'élément de pression (33) guidé en coulissement de la deuxième, respectivement de la première chambre de compensation (20, 11) sont accouplés l'un à l'autre par voie mécanique.

5. Dispositif selon l'une quelconque des revendications 1 à 4, **caractérisé en ce que**, en aval de la chambre tampon (23) montée dans le premier, respectivement le deuxième conduit de sortie (15, respectivement 22), on prévoit une soupape anti-retour (24) ou une soupape d'arrêt dans le premier, respectivement le deuxième conduit de sortie (15, 22).

## Claims

1. Device for the removal of toxic substances from blood with a balance chamber (14) which is divided by a flexible wall (13) into a first balance chamber half (12, 17) connected to a first inlet tube (9) and a second balance chamber half provided with a second inlet tube (18), where a first discharge tube (15) exits from the first balance chamber half (12) and a second discharge tube (22) exits from the second balance chamber half (17),
clamping devices (25 to 28) to close off the inlet tubes (9, 18) and the discharge tubes (15, 22),
a control unit (37) to activate the clamping devices (25 to 28), and
a first equalisation chamber (11) connected in line with the first inlet tube (9) and a second equalisation chamber (20) connected in line with the second inlet tube (18)
**characterised in that**
a buffer chamber with an adjustable volume (23) is provided connected in line with the first and / or second discharge tube (15, 22) of the balance chamber (14), whereby a coupling device is provided which is so designed that the volume of the buffer chamber (23) connected in line with the first or second discharge tube (15 or 22) can be changed in such a manner, in dependence upon the volume of the second or first equalisation chamber (20 or 11), that the volume of the buffer chamber is enlarged immediately before the emptying or during the emptying of the second or first equalisation chamber and is reduced during the filling of the second or first equalisation chamber.

2. Device in accordance with claim 1, **characterised in that** each of the first and second equalisation chambers (11, 20) is constantly kept under pressure from the compression devices (29, 30).

3. Device in accordance with claim 2, **characterised in that** each of the compression devices (29, 30) of the first and second equalisation chambers (11, 20) exhibit two pressure elements (31, 32; 33, 34) located over and / or under the chamber and which are pressed together by a spring element (35, 36) and that two pressure elements (38, 39) are provided which are located over and / or under the buffer chamber connected in line with the first or second discharge tubes (15 or 22) whereby the coupling device (60) couples together the pressure elements (38, 39) of the buffer chamber connected in line with the first or second discharge tube (15 or 22) and the pressure elements (31, 33) of the second or first equalisation chamber ( 20 or 11) in such a manner that the pressure elements (38, 39) move in opposite directions, whereby the volume of the buffer chamber (23) connected in line with the first or second discharge tube (15, 22) increases during the emptying of the second or first equalisation chamber (20 or 11) and decreases during the filling of the second or first equalisation chamber.

4. Device in accordance with claim 3, **characterised in that** one of the two pressure elements (31, 32, 38) of the first and second equalisation chambers (11, 20) and the buffer chamber connected in line with the first or second discharge tube (15, 22) are arranged in a fixed position and the other of the two pressure elements (33, 34, 39) are capable of displacement whereby the coupling device is a connection element (60) mounted between its two ends and capable of pivoting by means of which the displaceable pressure element (39) of the buffer chamber (23) connected in line with the first or second discharge tube (15 or 22) and the displaceable pressure element (33) of the second or first equalisation chamber (20, 11) are connected together mechanically.

5. Device in accordance with one of the claims 1 to 4, **characterised in that** a non-return valve or a stop valve (24) is provided in the first or second discharge tube (15, 22) downstream of the buffer chamber (23) connected in line with the first or second discharge tube (15, 22).
